# EUROPÄISCHE PATENTSCHRIFT

(11) **EP 3 140 642 B1**
(45) Veröffentlichungstag und Bekanntmachung des Hinweises auf die Patenterteilung: **05.04.2023**
(21) Anmeldenummer: 15733358.4
(22) Anmeldetag: 08.05.2015
(51) Int. Cl.: G01N 23/10, A61B 5/18, G08B 21/06, G08B 21/04, G06Q 50/26

(54) **VERFAHREN UND EINRICHTUNG ZUR ÜBERWACHUNG DER AUFMERKSAMKEIT EINER BEDIENPERSON**
METHOD AND DEVICE FOR MONITORING THE ATTENTIVENESS OF AN OPERATING PERSON
PROCÉDÉ ET DISPOSITIF POUR SURVEILLER L'ATTENTION D'UN OPÉRATEUR

(30) Priorität: 09.05.2014 DE 102014208711
(43) Veröffentlichungstag der Anmeldung: 15.03.2017
(73) Patentinhaber: Smiths Heimann GmbH, 65205 Wiesbaden (DE)
(72) Erfinder: RIEDEL, Ulrich, 55252 Mainz-Kastel (DE)
(74) Vertreter: Klunker IP Patentanwälte PartG mbB
(86) Internationale Anmeldenummer: PCT/EP2015/060214
(87) Internationale Veröffentlichungsnummer: WO 2015/169954

(56) Entgegenhaltungen:
- WO-A1-2013/187874
- US-A- 5 392 030
- US-A- 6 113 538
- US-A1- 2008 044 801
- US-A1- 2008 084 319
- US-A1- 2008 309 616
- US-B1- 6 899 540

## Beschreibung

Die Erfindung betrifft allgemein das Gebiet der Sicherheitseinrichtungen für Anlagen oder Maschinen, die bei ihrem Betrieb durch eine Bedienperson überwacht werden müssen. Im Besonderen betrifft die Erfindung ein Verfahren und eine Einrichtung zur Überwachung der Aufmerksamkeit einer Bedienperson.

### Hintergrund der Erfindung

Sicherheitseinrichtungen nach Art einer Totmannschaltung sind aus dem Stand der Technik hinreichend bekannt. Beispielsweise offenbart DE 10 2008 050 542 A1 einen Totmannschalter an einer Röntgenanlage, der aus Sicherheitsgründen bei einem Verstellen der Vorrichtung betätigt werden muss. Aus der DE 10 2013 207 143 A1 ist ein Berührungsbildschirm mit einer Druckerfassung als Bedienerschnittstelle für eine industrielle technische Einrichtung bekannt, die an einem Haltegriff ein Betätigungselement aufweist, das mit einer Auswerteeinrichtung signaltechnisch verbunden ist und ein Betätigen des Betätigungselements von der Auswerteeinrichtung zusätzlich zu einem Ausüben von Druck auf den Bereich des Berührungsbildschirms ausgewertet wird, damit beispielsweise ein Aktivieren von Antrieben nur dann erfolgt, wenn zusätzlich das Betätigungselement betätigt wird. Aus der DE 10 2005 030 934 B4 ist der Einsatz einer Drucktaste in einem Kraftfahrzeug bekannt, die von einem Fahrer periodisch oder auf Aufforderung betätigt werden muss, um Ermüden des Fahrers erkennen zu können.

Die bekannten Sicherheitseinrichtungen sind jedoch im Wesentlichen auf die Überprüfung der körperlichen Anwesenheit einer Bedienperson bzw. deren körperliche Unversehrtheit beschränkt. Gerade bei einer längeren Tätigkeit der Bedienperson an einer Anlage oder Maschine kann sich trotz in unregelmäßigen Zeitabständen angeforderten Betätigung der Sicherheitseinrichtung bei der Bedienperson eine gewissen Routine einstellen, die dazu führen kann, dass die Sicherheitseinrichtung von der Bedienperson unbewusst betätigt wird. Damit erfüllen die bekannten Sicherheitseinrichtungen zwar ihren primären Zweck, können jedoch die für sicherheitsrelevante Aspekte erforderliche Aufmerksamkeit der Bedienperson weder sicherstellen noch überprüfen.

WO 2013/187874 A1 offenbart ein interaktives Alarmierungssystem zum Sicherstellen, dass eine Person, wie z.B. ein Flugzeugführer, Lkw-Fahrer, Soldat oder Seemann auf Wache, oder Sicherheitspersonal, wachsam bleiben, d.h. nicht dösen, schlafen oder gar bewusstlos sind. Das Alarmierungssystem stellt einer Person Anweisungen und Zeit zur Verfügung, einen Code einzugeben, und alarmiert die Person oder Dritte, wenn der Code nicht rechtzeitig und richtig eingegeben wird. US 2003/0095046 A1 offenbart u.a. eine Vorrichtung und ein Verfahren zum Prüfen der Fähigkeiten eines Fahrers ein Fahrzeug zu führen, indem dem Fahrer auf einer Anzeige für eine bestimmte Zeit ein zufälliger Code angezeigt wird, den dieser innerhalb einer bestimmten Zeit korrekt eingeben muss. Ein ähnliches System offenbart die US 5 392 030 A.

US 2008/0044801 A1 und US 6 899 540 B1 zeigen jeweils mit der sogenannten Threat-Image-Projektion (TIP) Technologie ausgerüstete

Röntgeninspektionsanlagen, bei denen ein fiktiver Gegenstand in ein aktuelles zu inspizierendes Inspektionsobjekt hineinprojiziert werden kann, um die Aufmerksamkeit als auch Leistung einer Bedienperson zu Überwachen.

US 2008/309616 A1 zeigt Verfahren und Vorrichtung zum Erfassen der Wachsamkeit eines Gerätebedieners, indem ein sich bewegendes Symbol angezeigt wird und der Bediener aufgefordert wird, die Bewegungen des Symbols zu verfolgen, indem er ihm entweder mit den Augen auf einer am Kopf getragenen Anzeige oder mit einem Finger auf einem Touchscreen folgt.

US 6 113 538 A zeigt einen Spezialcomputer und ein Verfahren zum Testen der Wachsamkeit und geistigen Fitness von Benutzern vor Beginn der Arbeit oder einer potenziell gefährlichen Tätigkeit, wobei die Testverfahren Computerspielen ähnlich sind, aber eher die geistige Fitness und Wachsamkeit als die Eignung, Fähigkeit oder Intelligenz messen, wobei ein Basistest aus einer Vielzahl von Ja- oder Nein-Fragen, die auf grafischen Daten basieren, die dem Benutzer angezeigt werden, besteht.

US 2008/084319 A1 zeigt eine Vorrichtung, die eine unerwünschte Manipulation von Bedienelementen verhindert, indem sie die genannten Bedienelemente sperrt, bis eine Person einen Gedächtnistest besteht, der aus einem zufällig/pseudozufällig erzeugten Muster besteht.

### Offenbarung der Erfindung

Es ist eine Aufgabe der vorliegenden Erfindung, ein Verfahren und eine Vorrichtung vorzuschlagen, die neben der Überprüfung der Anwesenheit einer Bedienperson einer Röntgenprüfanlage zur zerstörungsfreien Inspektion von Inspektionsobjekten auch geeignet ist, deren Aufmerksamkeit zu überprüfen.

Die Aufgabe wird mit den Merkmalen der unabhängigen Ansprüche gelöst. Weitere Merkmale und Details der Erfindung ergeben sich aus den Unteransprüchen, der Beschreibung und den Zeichnungen. Dabei gelten Merkmale und Details, die in Zusammenhang mit dem erfindungsgemäßen Verfahren beschrieben werden, selbstverständlich auch in Zusammenhang mit der erfindungsgemäßen Vorrichtung und jeweils umgekehrt. Daher wird bezüglich der Offenbarung der einzelnen Aspekte wechselseitig Bezug genommen.

Ein Kerngedanke der Erfindung besteht darin, das aus dem Stand der Technik bekannte Prinzip einer Sicherheitseinrichtungen nach Art eines Totmannschalters so weiterzubilden, dass eine Betätigung der Sicherheitseinrichtung ein gewisses Maß an Aufmerksamkeit seitens der Bedienperson fordert. Der Erfinder schlägt im Wesentlichen vor, das Totmannschalterprinzip dahingehend weiterzubilden, dass die Betätigung der Sicherheitseinrichtung nicht zur Routine werden kann, indem bei einer Betätigung der Sicherheitseinrichtung eine zufällig erzeugte Information als eine Eingabeaufforderung seitens der Bedienperson berücksichtigt werden muss, damit die Betätigung seitens der Sicherheitseinrichtung als korrekte Antwort interpretiert wird. So wird verhindert, dass die Sicherheitseinrichtung quasi aus dem Unterbewusstsein routinemäßig betätigt werden kann. Bei dem erfindungsgemäßen Verfahren bzw. der erfindungsgemäßen Vorrichtung wird genau diese Schwachstelle der bekannten Sicherheitseinrichtungen überwunden.

Ein erster Aspekt der Erfindung betrifft ein Verfahren zur Überwachung der Aufmerksamkeit einer Bedienperson einer Röntgenprüfanlage zur zerstörungsfreien Inspektion von Inspektionsobjekten und zur Sicherstellung der Anwesenheit und/oder Aufmerksamkeit eines Bedieners der Röntgenprüfanlagemit gemäß Anspruch 1.

Dadurch, dass die Bedienperson nicht im Vorhinein die "richtige" Antwort bzw. Betätigung der Sicherheitseinrichtung kennen kann, wird sichergestellt, dass die Bedienperson mit der notwendigen Aufmerksamkeit die für die richtige Betätigung der Sicherheitseinrichtung angezeigte Information (bzw. Eingabeaufforderung) zur Kenntnis nehmen muss. Mit anderen Worten kennt die Bedienperson die "richtige" Antwort erst, wenn sie die angezeigte Information bewusst, d. h. aufmerksam, wahrgenommen hat.

Dabei ist es vorteilhaft, wenn festgestellt wird, ob eine aktuelle Ist-Eingabe durch die Bedienperson innerhalb eines vorbestimmten Zeitintervalls ab dem Anzeigen der Information erfolgt. Durch das vorbestimmte Zeitintervall, das ein Zeitfenster definiert, in dem die Betätigung durch die Bedienperson erfolgen muss, wird weiter ermöglicht, die Reaktionsschnelligkeit der Bedienperson als ein zusätzliches Maß für deren Aufmerksamkeit zu erfassen und zu berücksichtigen. Darüber hinaus legt das Zeitintervall eindeutig einen Zeitpunkt fest, ab dem auch keine Eingabe durch die Bedienperson als eine fehlerhafte Eingabe gewertet werden kann.

Erfindungsgemäß definiert die angezeigte Information einen zufällig bestimmten Ort auf einer Eingabeeinheit. Ein von der Bedienperson als Antwort berührter Ort an der Eingabeeinheit wird dann erfasst.

Basierend auf einem Vergleich des von der Bedienperson berührten Orts an der Eingabeeinheit als Ist-Eingabe mit dem zufällig bestimmten Ort als Soll-Eingabe kann dann das Signal, welches repräsentativ für die Aufmerksamkeit der Bedienperson ist erzeugt werden.

Erfindungsgemäß sind die Anzeigeeinheit und die Eingabeeinheit kombiniert in einem berührungsempfindlichen Bildschirm (Touchscreen).

Damit ist es möglich, auf dem berührungssensitiven Bildschirm (Touchscreen) einen zufällig bestimmten Ort zu markieren oder hervorzuheben, um den Betätigungsort auf der Anzeige als Soll-Eingabe zu definieren. D.h., der markierte Ort ist dann die Information (bzw. Eingabeaufforderung) Dies kann beispielsweise durch Anzeigen eines Kreuzes, eines Punkts, eines Kreises oder in irgendeiner anderen Weise graphisch auf dem Touchscreen erfolgen. Damit ist die Soll-Eingabe für die Bedienperson als Ort sichtbar auf der Anzeigeeinheit definiert. Anschließend, wie im Folgenden näher Beschrieben, muss zur Eingabe einer Antwort die Bedienperson lediglich den markierten Ort auf dem Touchscreen berühren. Damit ist die Ist-Eingabe festlegt.

Erfindungsgemäß wird dabei in einem ersten Zeitintervall der Bedienperson zunächst die zufällig bestimmte Information, welche die Soll-Eingabe definiert, angezeigt. Nach Ablauf des ersten Zeitintervalls wird die Information nicht mehr angezeigt und dann das Zeitintervall, in dem eine Eingabe der Bedienperson erfasst wird, gestartet. Bei dieser Ausführung wird die Aufmerksamkeit der Bedienperson noch mehr gefordert, da zum Zeitpunkt der Eingabe der Antwort die die "richtige" Antwort definierende Information (Eingabeaufforderung) bereits nicht mehr angezeigt wird. Die Soll-Eingabe muss quasi erinnert werden. Damit wird ausgeschlossen, dass das Berühren einer markierten Stelle bei der Bedienperson zur Routine wird.

Die vorstehenden optionalen Aspekte der Ausführungen können auch zufällig abwechselnd in einer Einrichtung kombiniert werden. Die damit geschaffene Abwechslung für die Bedienperson fördert ebenfalls die Aufmerksamkeit.

Besonders vorteilhaft bei einer Ausführung mit Rückmeldung ist, dass die Betätigung der Sicherheitseinrichtung durch die Bedienperson eine einen gewissen Zeitraum dauernde Interaktion der Bedienperson mit der Sicherheitseinrichtung erforderlich macht und damit noch besser sichergestellt werden kann, dass die Bedienperson ihre volle Aufmerksamkeit der Sicherheitseinrichtung und damit der Maschine oder Anlage widmet.

Auch bei dieser Ausführung kann mit zwei Zeitintervallen gearbeitet werden, wobei hier ein erstes Zeitintervall zunächst vorgesehen ist, in dem der Bedienperson eine Betätigung des Eingabeelements oder des Touchscreen ermöglicht wird. Der aktuelle durch die Betätigung erzeugte Ist-Wert wird der Bedienperson dabei bevorzugt quantitativ auf graphische und/oder alphanummerische Weise auf der Anzeigeeinheit oder dem Touchscreen angezeigt. Damit bekommt die Bedienperson die oben beschriebene Rückmeldung, anhand der sie die Eingabe korrigieren kann. In einem sich anschließenden Zeitintervall wird der Bedienperson ebenfalls bevorzugt optisch angezeigt, dass das zweite Zeitintervall begonnen hat, an dessen Ende die der aktuelle Ist-Wert gewertet, d.h. als Eingabe erfasst wird.

Bei allen beschriebenen Ausführungen ist es zusätzlich möglich, der Bedienperson neben der optischen Anzeige der zufällig bestimmten Information auch akustisch und/oder haptisch zu signalisieren, dass eine Überprüfung der Aufmerksamkeit erfolgt. Eine akustische Signalisierung kann beispielsweise mittels eines eindeutig der Funktion zugeordneten Geräuschs über einen Lautsprecher erfolgen. Eine haptische Signalisierung kann beispielsweise mittels einer Vibrationseinheit erfolgen, die beispielsweise in eine von der Bedienperson getragenen Kleidungsstück oder Armband etc. und/oder einer Sitzfläche integriert sein kann.

Ein zweiter Aspekt der Erfindung betrifft eine im Wesentlichen entsprechend dem vorstehend diskutierten Verfahren ausgebildete Einrichtung zur Überwachung der Aufmerksamkeit einer Bedienperson einer Röntgenprüfanlage zur zerstörungsfreien Inspektion von Inspektionsobjekten und zur Sicherstellung der Anwesenheit und/oder Aufmerksamkeit eines Bedieners der Röntgenprüfanlage gemäß dem Anspruch 3. Ähnlich wie bei dem Verfahren ist es vorteilhaft, wenn die Verarbeitungseinheit weiter eingerichtet ist, festzustellen, ob eine Ist-Eingabe innerhalb eines vorbestimmten Zeitintervalls ab dem Anzeigen der Information erfolgt ist.

Erfindungsgemäß ist die Verarbeitungseinheit eingerichtet, als die Information auf der Anzeigeeinheit einen zufällig bestimmten Ort auf der Eingabeeinheit zu markieren. Ein von der Bedienperson auf der Eingabeeinheit berührter Ort wird dann erfasst und als Ist-Eingabe mit dem angezeigten Ort als die Soll-Eingabe verglichen.

Wie bereits in Zusammenhang mit dem erfindungsgemäßen Verfahren erläutert, sind die Anzeigeeinheit und die Eingabeeinheit zu einem berührungssensitiven Bildschirm oder Berührungsbildschirm (Touchscreen) kombiniert.

Bevorzugt handelt es sich bei der Anzeigeeinheit um einen Bildschirm, der grundsätzlich jede Art von bekannter Anzeige sein kann, wie beispielsweise einem LCD-Bildschirm, einem TFT-Bildschirm oder Ähnlichem sein kann. Dabei ist die Anzeigeeinheit erfindungsgemäß ein Touchscreen.

Bevorzugt ist eine Ausführung der vorstehend beschriebenen Einrichtung mit einer Sicherheitseinrichtung einer Röntgenprüfanlage, deren zuverlässiger Betrieb nicht nur die Anwesenheit, sondern besonders die Aufmerksamkeit einer Bedienperson erfordert, gekoppelt, wobei die Sicherheitseinrichtung eingerichtet ist, die Anlage basierend auf dem Signal, welches repräsentativ für die Aufmerksamkeit der Bedienperson ist, zu steuern.

Beispielsweise kann die Sicherheitseinrichtung die Anlage in einen sicheren Zustand bringen, beispielsweise die Anlage ordentlich in einen vorbestimmten Zustand versetzen. Zusätzlich oder alternativ kann die Sicherheitseinrichtung einen Alarm auslösen, der zunächst die Aufmerksamkeit der Bedienperson erregen soll oder über entsprechende Kommunikationsverbindungen Dritte benachrichtigt und somit über den Fehlerzustand der Anlage informiert.

Die Erfindung eignet sich besonders gut zum Einsatz an Anlagen, deren weitgehend selbsttätige Funktion von einer Bedienperson beaufsichtigt oder überwacht wird. Die Erfindung kann aber grundsätzlich bei Anlagen eingesetzt werden, bei denen im Wesentlichen nicht nur die unversehrte Anwesenheit einer Bedienperson, sondern besonders deren Aufmerksamkeit bei der Überwachung/Beaufsichtigung der Maschine oder Anlage sichergestellt werden soll.

### Bevorzugte Ausführungsbeispiele

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Funktionsähnliche oder identische Bauteile oder Komponenten sind teilweise mit gleichen Bezugszeichen versehen. Die in der Beschreibung der Ausführungsbeispiele verwendeten Begriffe "links", "rechts", "oben" und "unten" beziehen sich auf die Zeichnungen in einer Ausrichtung mit normal lesbarer Figurenbezeichnung bzw. normal lesbaren Bezugszeichen. Die gezeigten und beschriebenen Ausführungsformen sind nicht als abschließend zu verstehen, sondern haben beispielhaften Charakter zur Erläuterung der Erfindung. Die detaillierte Beschreibung dient der Information des Fachmanns, daher werden bei der Beschreibung bekannte Schaltungen, Strukturen und Verfahren nicht im Detail gezeigt oder erläutert, um das Verständnis der vorliegenden Beschreibung nicht zu erschweren.

Figur 1 zeigt ein Ausführungsbeispiel der erfindungsgemäßen Überwachungseinrichtung mit einem Touchscreen als kombinierte Eingabe-/Ausgabeeinheit.

Figur 2 zeigt ein Flussdiagramm einer Ausführung des Überwachungsverfahrens.

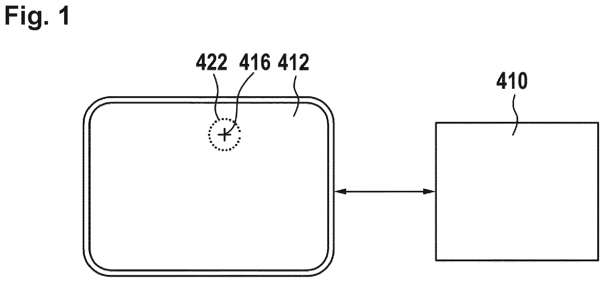

Figur 1 zeigt ein Ausführungsbeispiel einer erfindungsgemäßen Überwachungseinrichtung für die Aufmerksamkeit der Bedienperson. Die Verarbeitungseinheit 410 ist mit einem Touchscreen 412 als kombinierte Anzeige-/Eingabeeinheit operativ verbunden.

Zur Überwachung der Aufmerksamkeit der Bedienperson erzeugt die Verarbeitungseinheit 410 zunächst zufällig Koordinaten einer berührbaren Stelle des Touchscreens 412. Diese zufällig bestimmten Koordinaten definieren eine Stelle auf dem Touchscreen 412 als zufällig bestimmte Soll-Eingabe, die von einer Bedienperson zur Bestätigung ihrer Aufmerksamkeit berührt werden soll.

Zur Anzeige dieser zufällig bestimmten Soll-Eingabe an die Bedienperson, blendet die Verarbeitungseinheit 410 an der zufällig bestimmten Stelle des Touchscreens 412 als Soll-Eingabe ein Symbol 416 ein. Bei dem Symbol 416 handelt es sich im Ausführungsbeispiel um ein die Stelle markierendes Kreuz. Dabei kann es sich auch um ein anderes beliebiges Symbol, zum Beispiel einen Kreis, ein Viereck oder einfach einen Punkt handeln. Das Symbol sollte im Wesentlichen in der Lage sein, einen zu berührenden Ort auf dem Touchscreen mit der für das hier beschriebene Verfahren ausreichenden Genauigkeit zu markieren.

Ab dem Einblenden des Symbols 416 auf dem Touchscreen 412 wird von der Verarbeitungseinheit 410 wieder das Zeitfenster überwacht, innerhalb dessen die Bedienperson die auf dem Touchscreen 412 mit dem Symbol 416 markierte Stelle berühren soll.

Das Touchscreen 412 als gleichzeitige Anzeigeeinheit und Eingabeeinheit erfasst die Stelle, an der die Bedienperson das Touchscreen 412 berührt und meldet die Koordinaten der Berührstelle an die Verarbeitungseinheit 410 zurück. Die Verarbeitungseinheit 410 vergleicht die Koordinaten der Soll-Eingabe mit den Koordinaten mit der Ist-Eingabe. Basierend auf dem Vergleichsergebnis wird wieder das die Aufmerksamkeit der Bedienperson repräsentierende Signal erzeugt.

Da das Berühren einer Stelle auf einem Touchscreen 412 nicht einfach digital ausgewertet werden kann, wie beispielsweise das Betätigen einer mechanischen Taste, ist die Verarbeitungseinheit 410 eingerichtet, einen bestimmten Toleranzbereich beim Vergleich der Ist-Eingabe mit der Soll-Eingabe zu berücksichtigen.

Ein solcher Toleranzbereich ist in der Figur 4 durch den gestrichelten Kreis 422 um das Symbol 416 angedeutet. D. h., jede Berührung durch die Bedienperson innerhalb des durch den Kreis 422 markierten Bereichs auf dem Touchscreen 412 wird von der Verarbeitungseinheit 410 als der Soll-Eingabe entsprechend gewertet.

Im Ausführungsbeispiel der Figur1 ist es grundsätzlich nicht möglich, dass die Bedienperson digital unterscheidbar eine richtige oder eine falsche Eingabe vornimmt. Wie in Zusammenhang mit dem Ausführungsbeispiel bereits erläutert, kann dies durch einen Toleranzbereich, der sich durch Experimente ausreichend genau definieren lässt, aufgefangen werden.

Eine vorteilhafte Weiterbildung kann durch eine stärkere Rückmeldung von aktuellen Vergleichsergebnissen der aktuellen Ist-Eingabe mit der Soll-Eingabe verbessert werden.

Wenn die Bedienperson beim Eingeben ihrer Eingabe aufmerksam eine Rückkopplung der Verarbeitungseinheit wahrnimmt, kann sie innerhalb eines vorbestimmten Eingabezeitfensters ihre Eingabe anpassen. Beispielsweise kann eine solche Anpassung durch Verändern der Berührstelle des Fingers auf einem Touchscreen als Eingabeeinheit (vgl. Figur 1) erfolgen.

Mit einer solchen Korrekturmöglichkeit wird dem Zweck der Überwachungseinrichtung nicht geschadet, da die Berücksichtigung dieser Art von Rückmeldung der Bedienperson auch als Indikator für die Aufmerksamkeit der Bedienperson gewertet werden kann.

Eine Rückmeldung an die Bedienperson kann über die Anzeigeeinheit mittels eines intuitiv verständlichen Farbwechsels, beispielsweise von Rot nach Grün ggf. über Gelb in beliebigen Abstufungen als Indiz für die derzeitige Abweichung zwischen Soll-Eingabe und Ist-Eingabe erfolgen. Die Bedienperson kann dann basierend auf derart optisch rückgemeldeten Informationen die aktuelle Ist-Eingabe so weit anpassen, dass eine "richtige" Eingabe innerhalb des vorbestimmten Zeitfensters erreicht wird. Dies verhindert unnötige Fehlalarme bzw. hat eine motivierenden Einfluss auf die Bedienperson die eigene Ist-Eingabe gegebenenfalls zu korrigieren.

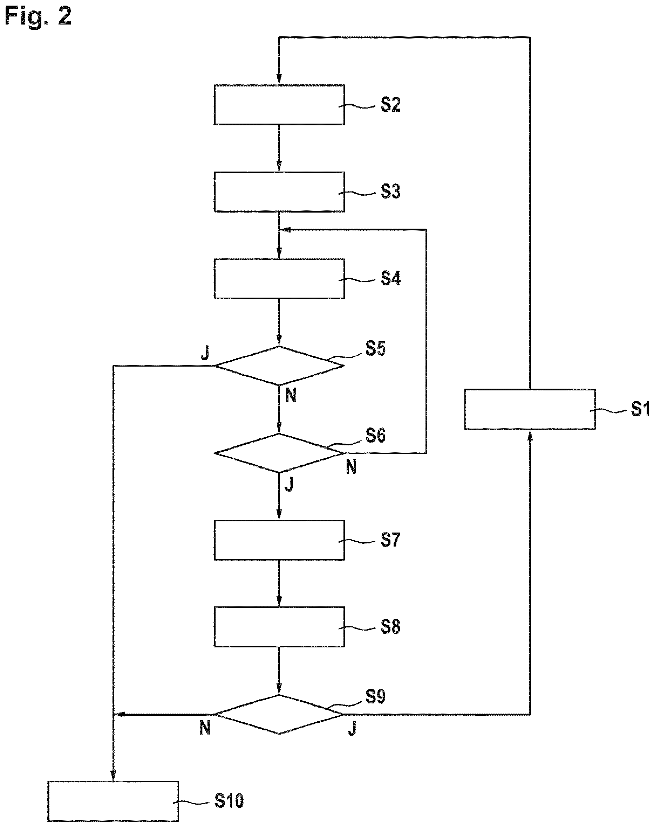

Figur 2 zeigt ein Flussdiagramm als mögliches Ausführungsbeispiel einer Implementierung des mittels der anhand der Figur 1 erläuterten Einrichtung implementierten Überwachungsverfahrens.

In einem Schritt S1 wird beispielsweise mittels eines Zufallsgenerators die Auslösung des Überwachungsverfahrens in unregelmäßigen Zeitabständen angestoßen. Dies kann beispielsweise durch einen zufallsgeneratorgesteuerten Zeitgeber (Timer) erfolgen, der nach Ablauf jeweils über einen Interrupt die Ausführung des Überwachungsverfahrens auslöst.

Sobald das Überwachungsverfahren durch den Timer in Schritt S1 ausgelöst wurde, geht es zu Schritt S2, in dem eine Information erzeugt wird, die eine zufällig bestimmte Soll-Eingabe für die Bedienperson definiert.

Danach geht das Verfahren zum Schritt S3, in dem die zufällig bestimmte Soll-Eingabe der Bedienperson angezeigt wird. Zusammen mit der Anzeige der Information an die Bedienperson wird ein weiterer vorbestimmter Timer gestartet, der das Zeitfenster definiert, innerhalb dessen eine Eingabe durch die Bedienperson erfolgen muss.

Nach dem Schritt S3 geht das Verfahren zum Schritt S4, in dem eine mögliche Eingabe seitens der Bedienperson an der Eingabeeinrichtung erfasst wird.

Vom Schritt S4 geht das Verfahren zum Schritt S5, in dem geprüft wird, ob der gestartete Timer für das Eingabezeitfenster bereits abgelaufen ist oder nicht. Falls der Timer bereits abgelaufen sein sollte, wird die Aufmerksamkeit der Bedienperson negativ bewertet und das Verfahren bricht an dieser Stelle ab und geht weiter zu Schritt S10, der weiter unten erläutert wird. Wenn der Timer noch nicht abgelaufen ist, geht das Verfahren von Schritt S5 weiter zu Schritt S6, in dem geprüft wird, ob eine Eingabe durch die Bedienperson erfolgt ist. Wenn keine Eingabe durch die Bedienperson erfolgt ist, geht das Verfahren von Schritt S6 zurück zum Schritt S4.

Wenn eine Eingabe durch die Bedienperson erfolgt ist, geht das Verfahren von Schritt S6 zum Schritt S7, in dem die erfasste Ist-Eingabe durch die Bedienperson als die Antwort auf die angezeigte Soll-Eingabe mit der Soll-Eingabe verglichen wird und ein Vergleichsergebnis erzeugt wird.

Danach geht das Verfahren von Schritt S7 zum Schritt S8, in dem basierend auf der Soll-Eingabe und der Ist-Eingabe, insbesondere dem Vergleichsergebnis, ein die Aufmerksamkeit der Bedienperson anzeigendes Signal zur weiteren Verwendung erzeugt wird.

Danach geht das Verfahren von Schritt S8 zum Schritt S9, in dem geprüft wird, ob die Bedienperson aufmerksam ist oder nicht. Wenn das die Aufmerksamkeit der Bedienperson anzeigende Signal anzeigt, dass die Bedienperson aufmerksam ist, geht das Verfahren von Schritt S9 wieder zurück zum Schritt S1 und wird von dort nach zufallsgesteuerter Auslösung erneut gestartet.

Wenn in Schritt S9 festgestellt wird, dass die Bedienperson nicht aufmerksam ist, geht das Verfahren von Schritt S9 zum Schritt S10. Im Schritt S10 bildet das die Aufmerksamkeit der Bedienperson anzeigende Signal die Grundlage für weitere Maßnahmen wie folgt.

Zunächst kann ein Alarm ausgelöst werden, der die Aufmerksamkeit der Bedienperson für die durch die Bedienperson zu überwachende Anlage oder Maschine wieder herstellen soll. Dabei ist es möglich, nach einem definierten Zeitfenster ab Alarmauslösung beispielsweise das Überwachungsverfahren erneut zu starten, um festzustellen, ob die Bedienperson nun wieder aufmerksam ist. Zusätzlich oder alternativ kann auch ein Alarm auf einer höheren Ebene ausgelöst werden, beispielsweise an einer der Bedienperson übergeordneten Stelle. Von dort können dann gegebenenfalls weitere Maßnahmen ergriffen werden.

Schließlich kann das die Aufmerksamkeit der Bedienperson anzeigende Signal auch für direkte Steuermaßnahmen an der Anlage oder Maschine verwendet werden. Beispielsweise ist es möglich, dass das die Aufmerksamkeit der Bedienperson anzeigende Signal über einen Signaleingang einer Steuereinrichtung der Anlage oder Maschine zugeführt wird, wobei die Steuereinrichtung im Falle der festgestellten Nichtaufmerksamkeit der Bedienperson die Anlage oder Maschine in einen vorbestimmten sicheren Zustand bringt.

Die vorstehend geschilderten Ausführungsbeispiele der Überwachungseinrichtung eignen sich besonders gut an solchen Anlagen oder Maschinen, bei denen die Aufmerksamkeit der Bedienperson u.a. für das Verarbeitungsergebnis der Maschine oder Anlage verantwortlich ist. Beispielsweise kann die Überwachungseinrichtung bzw. das Überwachungsverfahren an einer Röntgenprüfanlage zur zerstörungsfreien Inspektion von Objekten, wie beispielsweise Gepäckstücken an einem Flughafencheckpoint, eingesetzt werden, der kontinuierlich Gepäckstücke zugeführt werden und eine Bedienperson von dem Gepäckstück erzeugte Transmissionsbilder begutachten muss. Dabei ist die uneingeschränkte Aufmerksamkeit der Bedienperson erforderlich, da die Inspektion der Gepäckstücke nicht vollständig automatisiert stattfindet, sondern zu großen Teilen das Inspektionsergebnis auch von den eingebrachten Erfahrungen der Bedienperson abhängt. Die Aufmerksamkeit der Bedienperson kann mit dem vorstehend beschriebenen Überwachungseinrichtungen bzw. dem Überwachungsverfahren zufällig, aber regelmäßig überprüft werden.

## Patentansprüche

1. Verfahren zur Überwachung der Aufmerksamkeit einer Bedienperson einer Röntgenprüfanlage zur zerstörungsfreien Inspektion von Inspektionsobjekten und zur Sicherstellung der Anwesenheit und/oder Aufmerksamkeit einer Bedienperson der Röntgenprüfanlage, wobei das Verfahren die Schritten aufweist:
Anzeigen (S3) einer Information auf einem berührungssensitiven Bildschirm (412), die eine zufällig bestimmte Soll-Eingabe definiert;
Erfassen (S4) einer Ist-Eingabe der Bedienperson auf dem berührungssensitiven Bildschirm (412) als eine Antwort auf die angezeigte Soll-Eingabe; und
Bestimmen (S7) anhand der Soll-Eingabe und der Ist-Eingabe eines die Aufmerksamkeit der Bedienperson anzeigenden Signals; wobei
die Information einen zufällig bestimmten Ort auf dem berührungssensitiven Bildschirm (412) als Eingabeeinheit (412) definiert,
die Information als der zufällig bestimmter Ort auf dem berührungssensitiven Bildschirm (412) als Soll-Eingabe mittels einem Symbol (416) markiert angezeigt wird und
ein von der Bedienperson berührter Ort an der Eingabeeinheit (412) erfasst und als die Ist-Eingabe mit dem zufällig bestimmten Ort als Soll-Eingabe verglichen wird; und
wobei die Information in einem ersten Zeitintervall angezeigt wird und nach Ablauf des ersten Zeitintervalls nicht mehr angezeigt wird, wobei nach Ablauf des ersten Zeitintervalls ein zweites Zeitintervall gestartet wird, in dem die Ist-Eingabe der Bedienperson erfasst wird.

2. Verfahren nach Anspruch 1, weiter mit:
Feststellen (S5), ob eine Ist-Eingabe innerhalb eines vorbestimmten Zeitintervalls ab dem Anzeigen der Soll-Eingabe erfolgt.

3. Einrichtung zur Überwachung der Aufmerksamkeit einer Bedienperson einer Röntgenprüfanlage zur zerstörungsfreien Inspektion von Inspektionsobjekten und zur Sicherstellung der Anwesenheit und/oder Aufmerksamkeit einer Bedienperson der Röntgenprüfanlage, wobei die Vorrichtung aufweist:
eine Verarbeitungseinheit (410), die zum Erzeugen einer Information, die eine zufällig bestimmte Soll-Eingabe definiert, konfiguriert ist;
eine Anzeigeeinheit (412) zum Anzeigen der Information; und
eine Eingabeeinheit (412) zum Erfassen einer Ist-Eingabe der Bedienperson als eine Antwort auf die angezeigte Soll-Eingabe; wobei die Verarbeitungseinheit (410) mit der Anzeigeeinheit (412) und der Eingabeeinheit (412) operativ gekoppelt ist und zum Bestimmen eines die Aufmerksamkeit der Bedienperson anzeigenden Signals basierend auf der Soll-Eingabe und der Ist-Eingabe eingerichtet ist; wobei
die Anzeigeeinheit und die Eingabeeinheit in einem berührungssensitiven Bildschirm (412) kombiniert sind, und
die Verarbeitungseinheit (410) eingerichtet ist,
als die Information auf der Anzeigeeinheit (412) einen zufällig bestimmten Ort auf dem berührungssensitiven Bildschirm (412) als Eingabeeinheit (412) durch Markieren mit einem Symbol anzuzeigen,
einen von der Bedienperson auf der Eingabeeinheit (412) berührten Ort zu erfassen und als Ist-Eingabe mit dem angezeigten Ort als Soll-Eingabe zu vergleichen,
die Information in einem ersten Zeitintervall anzuzeigen und nach Ablauf des ersten Zeitintervalls nicht mehr anzuzeigen und
nach Ablauf des ersten Zeitintervalls ein zweites Zeitintervall zu starten, in dem die Ist-Eingabe der Bedienperson erfasst wird.

4. Einrichtung nach Anspruch 3, wobei die Verarbeitungseinheit (410) eingerichtet ist, festzustellen, ob die Ist-Eingabe innerhalb eines vorbestimmten Zeitintervalls ab dem Anzeigen der Soll-Eingabe erfolgt.

5. Einrichtung nach einem der Ansprüche 3 oder 4, wobei die Einrichtung mit einer Sicherheitseinrichtung der Röntgenprüfanlage, deren zuverlässiger Betrieb, die Anwesenheit und/oder Aufmerksamkeit der Bedienperson erfordert, gekoppelt ist, die eingerichtet ist, die Anlage basierend auf dem Signal für die Anwesenheit und/oder Aufmerksamkeit der Bedienperson zu steuern.

## Claims

1. Method for monitoring the attention of an operator of an X-ray inspection apparatus for non-destructive inspection of inspection objects and for ensuring the presence and/or attention of an operator of the X-ray inspection apparatus, the method comprising the steps of:
displaying (S3), on a touch-sensitive screen (412), information defining a randomly determined set input;
detecting (S4) an operator's as-is input on the touch-sensitive screen (412) as a response to the displayed set input; and
determining (S7), with reference to the set input and the as-is input, a signal indicative of the operator's attention; wherein
the information defines a randomly determined location on the touch-sensitive screen (412) as the input unit (412),
the information is displayed as the randomly determined location on the touch-sensitive screen (412) as a set input marked by a symbol (416), and
a location touched by the operator is detected on the input unit (412) and compared as the as-is input with the randomly determined location as the set input; and
wherein the information is displayed in a first time interval and is no longer displayed after the first time interval has elapsed, wherein after the first time interval has elapsed, a second time interval is started in which the as-is input of the operator is detected.

2. Method according to claim 1, further comprising:
determining (S5) whether an as-is input occurs within a predetermined time interval from when the set input is displayed.

3. Apparatus for monitoring the attention of an operator of an X-ray inspection apparatus for non-destructive inspection of inspection objects and for ensuring the presence and/or attention of an operator of the X-ray inspection apparatus, the apparatus comprising:
a processing unit (410) configured to generate information defining a randomly determined set input;
a display unit (412) for displaying the information; and
an input unit (412) for detecting an as-is input from the operator as a response to the displayed set input; wherein the processing unit (410) is operatively coupled to the display unit (412) and the input unit (412) and is configured to determine a signal indicative of the operator's attention based on the set input and the as-is input; wherein
the display unit and the input unit are combined in a touch-sensitive screen (412), and
the processing unit (410) is configured
to display as the information on the display unit (412) a randomly determined location on the touch-sensitive screen (412) as the input unit (412) by marking it with an icon,
to detect a location touched by the operator on the input unit (412) and to compare it as-is input with the displayed location as set input, to display the information in a first time interval and to stop displaying it after the first time interval has elapsed; and
after expiration of the first time interval, to start a second time interval in which the as-is input of the operator is detected.

4. Apparatus according to claim 3, wherein the processing unit (410) is configured to determine whether the as-is input occurs within a predetermined time interval from displaying the set input.

5. Apparatus according to any one of claims 3 or 4, wherein the device is coupled to a safety device of the X-ray inspection apparatus, the reliable operation of which requires the presence and/or attention of the operator, which is configured to control the device based on the signal for the presence and/or attention of the operator.

## Revendications

1. Procédé de surveillance de l'attention d'un opérateur d'une installation de vérification aux rayons X destinée à l'inspection non destructive d'objets d'inspection et à la garantie de la présence et/ou de l'attention d'un opérateur de l'installation de vérification aux rayons X, cependant que le procédé comprend les étapes :
affichage (S3) d'une information définissant une entrée prescrite, déterminée aléatoirement, sur un écran (412) sensible au toucher ;
saisie (S4) d'une entrée réelle de l'opérateur sur l'écran (412) sensible au toucher en tant qu'une réponse à l'entrée prescrite affichée ; et
détermination (S7), au moyen de l'entrée prescrite et de l'entrée réelle, d'un signal indiquant l'attention de l'opérateur, cependant que
l'information définit un emplacement déterminé aléatoirement sur l'écran (412) sensible au toucher en tant qu'unité d'entrée (412),
l'information en tant que l' emplacement déterminé aléatoirement sur l'écran (412) sensible au toucher est affichée de façon marquée au moyen d'un symbole (416) en tant qu'entrée prescrite, et
un emplacement touché par l'opérateur est saisi à l'unité d'entrée (412) et est comparé en tant que l'entrée réelle avec l' emplacement déterminé aléatoirement en tant qu'entrée prescrite ; et
cependant que l'information est affichée dans un premier intervalle de temps et n'est plus affichée après écoulement du premier intervalle de temps, cependant que, après écoulement du premier intervalle de temps, un deuxième intervalle de temps est démarré, dans lequel l'entrée réelle de l'opérateur est saisie.

2. Procédé selon la revendication 1, comprenant en outre :
constatation (S5) de si une entrée réelle a lieu à l'intérieur d'un intervalle de temps prédéterminé à partir de l'affichage de l'entrée prescrite.

3. Equipement de surveillance de l'attention d'un opérateur d'une installation de vérification aux rayons X destinée à l'inspection non destructive d'objets d'inspection et d'assurance de la présence et/ou de l'attention d'un opérateur de l'installation de vérification aux rayons X, cependant que le dispositif comporte :
une unité de traitement (410) configurée pour la génération d'une information définissant une entrée prescrite déterminée aléatoirement,
une unité d'affichage (412) pour l'affichage de l'information ; et
une unité d'entrée (412) pour la saisie d'une entrée réelle de l'opérateur en tant qu'une réponse à l'entrée prescrite affichée ; cependant que l'unité de traitement (410) est couplée opérationnellement à l'unité d'affichage (412) et à l'unité d'entrée (412) et est équipée pour la détermination d'un signal, basé sur l'entrée prescrite et l'entrée réelle, indiquant l'attention de l'opérateur, cependant que
l'unité d'affichage et l'unité d'entrée sont combinées dans un écran (412) sensible au toucher, et
l'unité de traitement (410) est équipée pour
en tant que l'information sur l'unité d'affichage (412), afficher par marquage avec un symbole un emplacement déterminé aléatoirement sur l'écran (412) sensible au toucher en tant qu'unité d'entrée (412),
saisir un emplacement touché par l'opérateur sur l'unité d'entrée (412) et le comparer en tant qu'entrée réelle avec l' emplacement affiché en tant qu'entrée prescrite,
afficher l'information dans un premier intervalle de temps et ne plus l'afficher après écoulement du premier intervalle de temps, et
après écoulement du premier intervalle de temps, démarrer un deuxième intervalle de temps dans lequel l'entrée réelle de l'opérateur est saisie.

4. Equipement selon la revendication 3, cependant que l'unité de traitement (410) est équipée pour constater si l'entrée réelle a lieu à l'intérieur d'un intervalle de temps prédéterminé à partir de l'affichage de l'entrée prescrite.

5. Equipement selon une des revendications 3 ou 4, cependant que l'équipement est couplé à un équipement de sécurité, de l'installation de vérification aux rayons X, dont le fonctionnement fiable nécessite la présence et/ou l'attention de l'opérateur, qui est équipé pour commander l'installation sur la base du signal de la présence et/ou de l'attention de l'opérateur.
